# EUROPEAN PATENT SPECIFICATION

(11) **EP 4 233 684 B1**
(45) Date of publication and mention of the grant of the patent: **19.08.2026**
(21) Application number: 21929209.1
(22) Date of filing: 14.12.2021
(51) Int. Cl.: G06T 7/00, A61B 1/00

(54) **PROGRAM, INFORMATION PROCESSING METHOD, AND INFORMATION PROCESSING DEVICE**
PROGRAMM, INFORMATIONSVERARBEITUNGSVERFAHREN UND INFORMATIONSVERARBEITUNGSVORRICHTUNG
PROGRAMME, MÉTHODE DE TRAITEMENT D'INFORMATIONS ET DISPOSITIF DE TRAITEMENT D'INFORMATIONS

(30) Priority: 04.03.2021 JP 2021034569
(43) Date of publication of application: 30.08.2023
(73) Proprietor: HOYA CORPORATION, Shinjuku-ku Tokyo 160-8347 (JP)
(72) Inventor: MATSUMURA, Kyosuke, Tokyo 160-8347 (JP)
(74) Representative: Lucke, Andreas
(86) International application number: PCT/JP2021/045921
(87) International publication number: WO 2022/185651

(56) References cited:
- WO-A1-2019/088121
- WO-A1-2019/088121
- WO-A1-2020/121906
- WO-A1-2020/121906
- US-A1- 2018 225 820
- US-A1- 2018 225 820
- ZHU RONGSHENG ET AL: "Lesion detection of endoscopy images based on convolutional neural network features", 2015 8TH INTERNATIONAL CONGRESS ON IMAGE AND SIGNAL PROCESSING (CISP), IEEE, 14 October 2015 (2015-10-14), pages 372 - 376, XP032867115, DOI: 10.1109/CISP.2015.7407907

## Description

### Technical Field

The present technology relates to a program, an information processing method, and an information processing device.

### Background Art

Computer-aided diagnosis technology has been developed which automatically detects lesions using a learning model from medical images such as endoscopic images. A method of generating a learning model by supervised machine learning using training data with a correct answer label is known. A learning model is disclosed which learns by a learning method of combining first learning using an image group captured by a normal endoscope as the training data and second learning using an image group captured by a capsule endoscope as the training data (for example, WO 2017/175282 A). WO 2019/088121 A1 discloses an image diagnosis assistance apparatus, a data collection method, an image diagnosis assistance method and an image diagnosis assistance program. The image diagnosis assistance apparatus is provided with: a lesion assessment unit that assesses, by a convolutional neural network, the denomination and the position of a lesion which is present in a digestive system endoscopic image of a patient captured by a digestive system endoscopic imaging device and information about accuracies thereof; and a display control unit that performs control for generating an analysis result image in which the denomination and the position of the lesion and the accuracies thereof are displayed and for displaying the image on the digestive system endoscopic image. In the convolutional neural network, learning processing is performed on the basis of the denominations and the positions of the lesions that are present in a plurality of digestive system tumor endoscopic images predetermined by feature extraction of conditions of atrophy, intestinal metaplasia, swelling or depression of a mucous membrane, and mucous membrane color tone.

### Summary of Invention

### Technical Problem

However, the computer-aided diagnosis technology described in WO 2017/175282 A has a problem that, in a case where a region of interest (ROI) is included in an input image, diagnosis support from the viewpoint of enlarging and displaying the region of interest is not considered.

In one aspect, an object is to provide a program or the like that efficiently performs processing of enlarging and displaying a region of interest in a case where the region of interest is included in an endoscopic image.

### Solution to Problem

The invention is defined by the appended claims.

A program according to an aspect of the present disclosure causes a computer to perform processing of acquiring an image captured by an endoscope, in a case where the image captured by the endoscope is input, inputting the acquired image to a learned model learned to output a position of a region of interest included in the image, acquiring a position of a region of interest included in the acquired image from the learned model, and outputting an enlarged image in which a portion of the image including the region of interest is enlarged on the basis of the acquired position of the region of interest.

An information processing method according to an aspect of the present disclosure causes a computer to perform processing of acquiring an image captured by an endoscope, in a case where the image captured by the endoscope is input, inputting the acquired image to a learned model learned to output a position of a region of interest included in the image, acquiring a position of a region of interest included in the acquired image from the learned model, and outputting an enlarged image in which a portion of the image including the region of interest is enlarged on the basis of the acquired position of the region of interest.

An information processing device according to an aspect of the present disclosure including an image acquisition unit that acquires an image captured by an endoscope, an input unit that, in a case where the image captured by the endoscope is input, inputs the acquired image to a learned model learned to output a position of a region of interest included in the image, a position acquisition unit that acquires a position of a region of interest included in the acquired image from the learned model, and an output unit that outputs an enlarged image in which a portion of the image including the region of interest is enlarged on the basis of the acquired position of the region of interest.

### Advantageous Effects of Invention

According to the present disclosure, it is possible to provide the program or the like that efficiently performs the processing of enlarging and displaying the region of interest in a case where the region of interest is included in an endoscopic image.

### Brief Description of Drawings

Fig. 1 is a schematic diagram illustrating an outline of an endoscope system according to a first embodiment.
Fig. 2 is a block diagram illustrating a configuration example of an endoscope device included in the endoscope system.
Fig. 3 is a block diagram illustrating a configuration example of an information processing device included in the endoscope system.
Fig. 4 is a functional block diagram exemplifying functional units included in a control unit of the information processing device.
Fig. 5 is an explanatory diagram related to a learned model (region-of-interest learning model).
Fig. 6 is an explanatory diagram illustrating an aspect of a display screen of an enlarged image.
Fig. 7 is a flowchart illustrating an example of a processing procedure performed by the control unit of the information processing device.
Fig. 8 is an explanatory diagram illustrating an aspect of a display screen of an enlarged image according to a second embodiment (a plurality of regions of interest).
Fig. 9 is a flowchart illustrating an example of a processing procedure performed by the control unit of the information processing device.
Fig. 10 is a functional block diagram exemplifying functional units included in a control unit of an information processing device according to a third embodiment (second learned model).
Fig. 11 is an explanatory diagram related to the second learned model (diagnosis support learning model).
Fig. 12 is a flowchart illustrating an example of a processing procedure performed by the control unit of the information processing device.

### Description of Embodiments

### (First Embodiment)

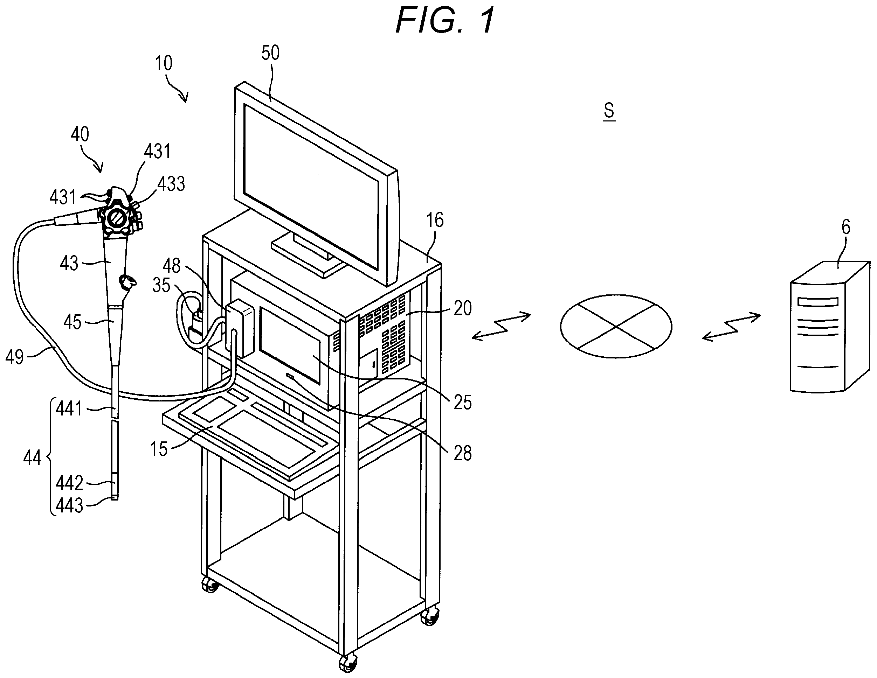

Hereinafter, the present invention will be specifically described with reference to the drawings illustrating embodiments of the present invention. Fig. 1 is a schematic diagram illustrating an outline of a diagnosis support system S according to a first embodiment. The diagnosis support system S includes an endoscope device 10 and an information processing device 6 communicatively connected to the endoscope device 10.

The endoscope device 10 transmits an image (a captured image) captured by an image sensor of an endoscope 40 to a processor 20 for an endoscope, and the processor 20 for an endoscope performs various types of image processing such as gamma correction, white balance correction, and shading correction, thereby generating an endoscopic image set to be easily observed by an operator. The endoscope device 10 outputs (transmits) the generated endoscopic image to the information processing device 6. When acquiring the endoscopic image transmitted from the endoscope device 10, the information processing device 6 performs various types of information processing on the basis of the endoscopic image, extracts information (region-of-interest information) related to a region of interest (ROI) included in the endoscopic image, generates an enlarged image of the region of interest on the basis of the region of interest information, and outputs the enlarged image to the endoscope device 10 (the processor 20 for an endoscope). The region of interest (ROI) refers to as a region of interest to a doctor or the like who is an operator of the endoscope 40, and is, for example, a region where a lesion, a lesion candidate, a drug, a treatment tool, a marker, and the like are located (present). The enlarged image of the region of interest output from the information processing device 6 is displayed on a display device 50 connected to the endoscope device 10.

The endoscope device 10 includes the processor 20 for an endoscope, the endoscope 40, and the display device 50. The display device 50 is, for example, a liquid crystal display device or an organic electro luminescence (EL) display device.

The display device 50 is provided on an upper shelf of a storage rack 16 with casters. The processor 20 for an endoscope is stored in a middle shelf of the storage rack 16. The storage rack 16 is disposed in the vicinity of a bed for endoscopic examination (not illustrated). The storage rack 16 includes a pull-out shelf on which a keyboard 15 connected to the processor 20 for an endoscope is provided.

The processor 20 for an endoscope has a substantially rectangular parallelepiped shape and includes a touch panel 25 on one surface. A reading unit 28 is disposed below the touch panel 25. The reading unit 28 is a connection interface for performing reading and writing on a portable recording medium such as a USB connector, a secure digital (SD) card slot, a compact disc read only memory (CD-ROM) drive, or the like.

The endoscope 40 includes an insertion portion 44, an operation unit 43, a universal cord 49, and a scope connector 48. The operation unit 43 includes a control button 431. The insertion portion 44 is long and has one end connected to the operation unit 43 via a bend preventing portion 45. The insertion portion 44 includes a soft portion 441, a bending section 442, and a distal tip portion 443 in order from the side of the operation unit 43. The bending section 442 is bent based on an operation of a bending knob 433. Physical detection devices such as a three-axis acceleration sensor, a gyro sensor, a geomagnetic sensor, a magnetic coil sensor, and an endoscope-insertion-type observation device (colonoscope navigation) may be attached to the insertion portion 44. In a case where the endoscope 40 is inserted into a body of a subject, detection results from these physical detection devices may be acquired.

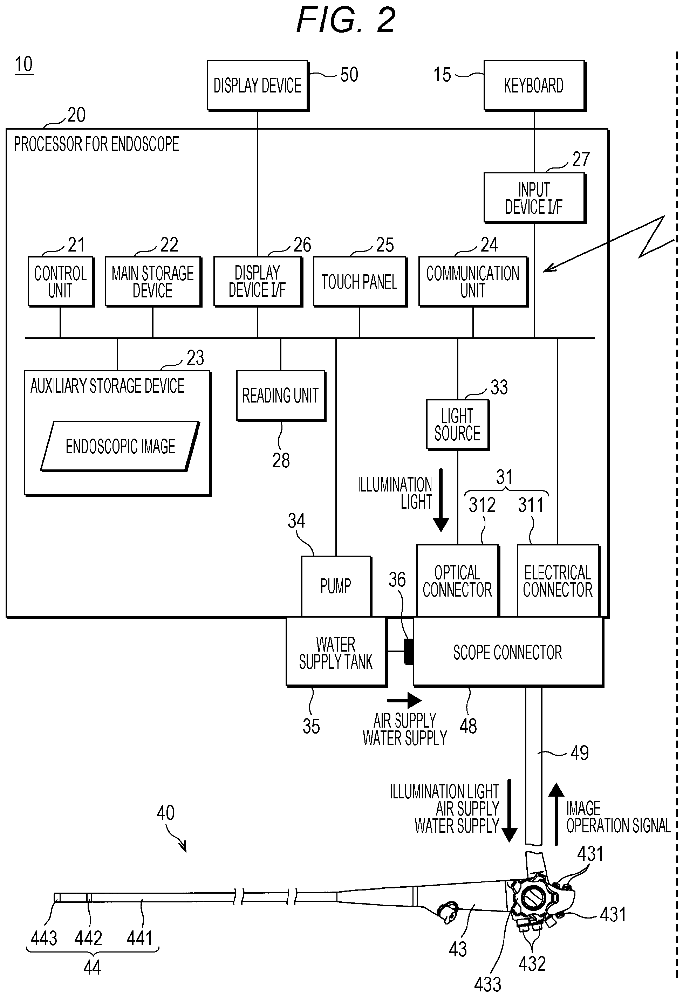

The universal cord 49 is long and has a first end connected to the operation unit 43 and a second end connected to the scope connector 48. The universal cord 49 is soft. The scope connector 48 has a substantially rectangular parallelepiped shape. The scope connector 48 includes an air/water supply port 36 (see Fig. 2) for connecting an air/water supply tube.

Fig. 2 is a block diagram illustrating a configuration example of the endoscope device 10 included in the diagnosis support system S. A control unit 21 is an arithmetic control device that executes a program according to the present embodiment. One or a plurality of central processing units (CPUs), graphics processing units (GPUs), multicore CPUs, or the like is used for the control unit 21. The control unit 21 is connected to each hardware unit constituting the processor 20 for an endoscope via a bus.

A main storage device 22 is, for example, a storage device such as a static random access memory (SRAM), a dynamic random access memory (DRAM), or a flash memory. The main storage device 22 temporarily stores information necessary in the middle of processing performed by the control unit 21 and a program being executed by the control unit 21. An auxiliary storage device 23 is, for example, a storage device such as an SRAM, a flash memory, or a hard disk and is a storage device with a larger capacity than the main storage device 22. In the auxiliary storage device 23, for example, the acquired captured image and the generated endoscopic image may be stored as intermediate data.

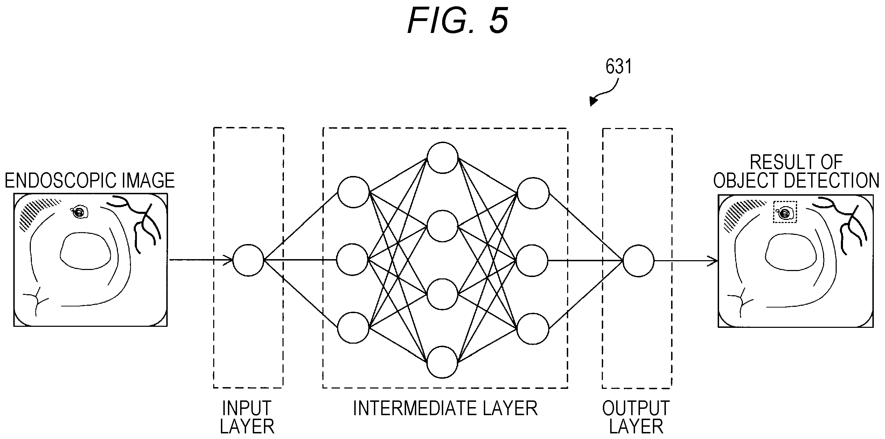

A communication unit 24 is a communication module or a communication interface for communicating with the information processing device 6 via a network in a wired or wireless manner and is, for example, a narrow-area wireless communication module such as Wi-Fi (registered trademark) or Bluetooth (registered trademark) or a wide-area wireless communication module such as 4G, Long Term Evolution (LTE), or 5G. The touch panel 25 includes a display unit such as a liquid crystal display panel and an input unit layered on the display unit. The communication unit 24 may communicate with a CT device, an MRI device (see Fig. 5), or a storage device (not illustrated) that stores data output from these devices.

A display device I/F 26 is an interface for connecting the processor 20 for an endoscope and the display device 50. An input device I/F 27 is an interface for connecting the processor 20 for an endoscope and an input device such as the keyboard 15.

A light source 33 includes, for example, a high-luminance white light source such as a white LED or a xenon lamp, and a special light source using a special light LED such as a narrow-band light LED that emits narrow-band light. The light source 33 is connected to the bus via a driver (not illustrated). Turning on and off of the light source 33 and the change of luminance are controlled by the control unit 21. Illumination light emitted from the light source 33 is incident on an optical connector 312. The optical connector 312 engages with the scope connector 48 to supply the illumination light to the endoscope 40.

A pump 34 generates a pressure for the air supply and water supply function of the endoscope 40. The pump 34 is connected to the bus via a driver (not illustrated). Turning on and off of the pump 34 and the change of pressure are controlled by the control unit 21. The pump 34 is connected to the air/water supply port 36 provided in the scope connector 48 via a water supply tank 35.

An outline of functions of the endoscope 40 connected to the processor 20 for an endoscope will be described. A fiber bundle, a cable bundle, an air supply tube, a water supply tube, and the like are inserted inside the scope connector 48, the universal cord 49, the operation unit 43, and the insertion portion 44. The illumination light emitted from the light source 33 is emitted from an illumination window provided at the distal tip portion 443 via the optical connector 312 and the fiber bundle. The image sensor provided at the distal tip portion 443 captures a range illuminated by the illumination light. The captured image is transmitted from the image sensor to the processor 20 for an endoscope via the cable bundle and an electrical connector 311.

The control unit 21 of the processor 20 for an endoscope functions as an image processing unit 211 by executing a program stored in the main storage device 22. The image processing unit 211 performs various types of image processing such as gamma correction, white balance correction, and shading correction on an image (a captured image) output from the endoscope 40 and outputs the image as an endoscopic image.

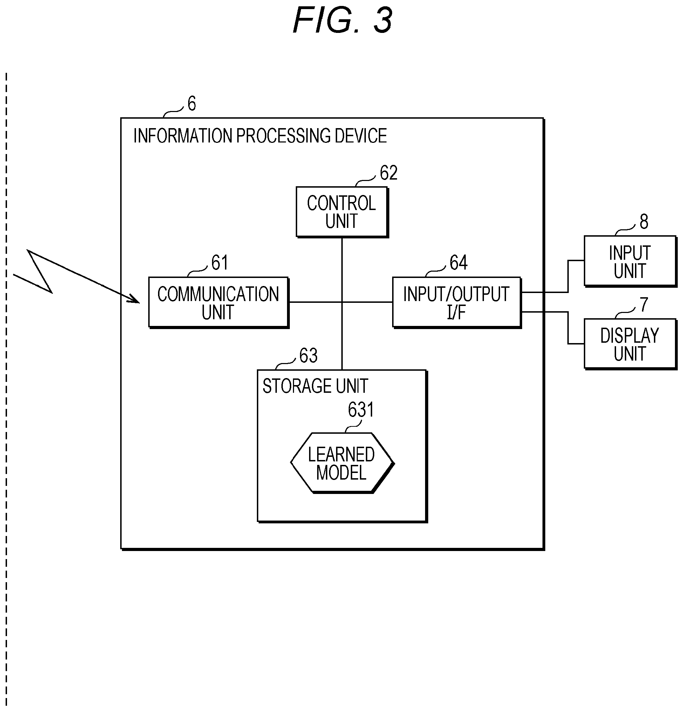

Fig. 3 is a block diagram illustrating a configuration example of the information processing device 6 included in the diagnosis support system S. The information processing device 6 includes a control unit 62, a communication unit 61, a storage unit 63, and an input/output I/F 64. The information processing device 6 is, for example, a server device, a personal computer, or the like. The server device includes not only a single server device but also a cloud server device or a virtual server device including a plurality of computers. The information processing device 6 may be provided as a cloud server located on an external network accessible from the processor 20 for an endoscope.

The control unit 62 includes one or a plurality of arithmetic processing devices having a time counting function, such as central processing units (CPUs), microprocessing units (MPUs), and graphics processing units (GPUs), and performs various types of information processing, control processing, and the like related to the information processing device 6 by reading and executing a program stored in the storage unit 63.

The storage unit 63 includes a volatile storage area such as a static random access memory (SRAM), a dynamic random access memory (DRAM), or a flash memory and a nonvolatile storage area such as an EEPROM or a hard disk. The storage unit 63 stores in advance a program and data to be referred to at the time of processing. The program stored in the storage unit 63 may be a program that is read from a recording medium readable by the information processing device 6. In addition, the program may be a program that is downloaded from an external computer (not illustrated) connected to a communication network (not illustrated) and is stored in the storage unit 63.

The storage unit 63 stores an entity file (an instance file of a neural network (NN)) constituting a learned model 631 (a region-of-interest learning model) or the like to be described later. These entity files may be configured as a part of the program. The storage unit 63 further stores various predetermined set values (preset data) for generating and outputting (displaying) an enlarged image. The preset data may include, for example, a set value (a preset value) for determining an output mode (a main screen or a sub-screen) of the enlarged image, an enlargement ratio at the time of enlargement and display (outputting the enlarged image), a flag value as to whether or not to change to a special light observation mode at the time of enlargement and display, and a predetermined value (an accuracy probability threshold) for dividing the accuracy probability of the region of interest. Details of the preset data will be described later.

The communication unit 61 is a communication module or a communication interface for communicating with the endoscope device 10 in a wired or wireless manner and is, for example, a narrow-area wireless communication module such as Wi-Fi (registered trademark) or Bluetooth (registered trademark) or a wide-area wireless communication module such as 4G, LTE, or 5G.

The input/output I/F 64 is compliant with a communication standard such as USB or DSUB, and is a communication interface for performing serial communication with an external device connected to the input/output I/F 64. For example, a display unit 7 such as a display and an input unit 8 such as a mouse or a keyboard are connected to the input/output I/F 64, and the control unit 62 outputs, to the display unit 7, a result of information processing performed on the basis of an execution command or an event input from the input unit 8.

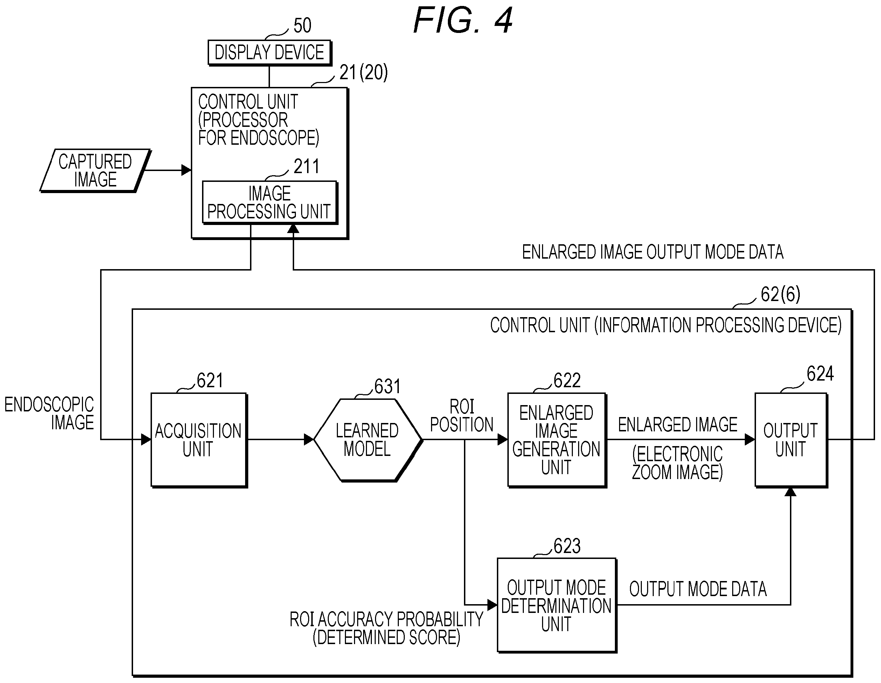

Fig. 4 is a functional block diagram exemplifying functional units included in the control unit 62 of the information processing device 6. The control unit 21 of the processor 20 for an endoscope (the endoscope device 10) executes the program stored in the main storage device 22, thereby functioning as the image processing unit 211. The image processing unit 211 of the processor 20 for an endoscope performs various types of image processing such as gamma correction, white balance correction, and shading correction on the image (the captured image) output from the endoscope, and outputs the image as the endoscopic image. The processor 20 for an endoscope further acquires the enlarged image and output mode data when the enlarged image is output, which are output from the information processing device 6, and outputs the enlarged image to the display device 50 on the basis of the output mode data.

The control unit 62 of the information processing device 6 functions as an acquisition unit 621, the learned model 631, an enlarged image generation unit 622, an output mode determination unit 623, and an output unit 624 by executing the program stored in the storage unit 63.

The acquisition unit 621 acquires the endoscopic image output by the processor 20 for an endoscope. The acquisition unit 621 inputs the acquired endoscopic image to the learned model 631 (region-of-interest learning model). In a case where the endoscopic image is output (transmitted) from the processor 20 for an endoscope to the information processing device 6 as a video, the acquisition unit 621 may acquire the endoscopic image in units of frames in the video and input the endoscopic image to the learned model 631.

The learned model 631 acquires and inputs the endoscopic image output from the acquisition unit 621, and outputs information related to the region of interest included in the input endoscopic image. The information related to the region of interest includes a position indicating a portion of the region of interest included in the endoscopic image, and an accuracy probability when the region of interest is estimated. The position indicating the portion of the region of interest included in the endoscopic image includes, for example, coordinates of two points in the image coordinate system of the endoscopic image, and the position indicating the portion of the region of interest is specified by a rectangular frame (a bounding box) with the two points as diagonal points. The accuracy establishment when the region of interest is estimated is, for example, a value of a class probability (an estimated score) indicating the estimation accuracy of the region of interest extracted from the endoscopic image, and is indicated by a value of 0 to 1, for example. In this case, a value closer to 1 indicates that the region of interest extracted from the endoscopic image is a true region of interest (the estimation accuracy is higher). As described above, the learned model 631 outputs the position and the accuracy probability as the information related to the region of interest in a case where the region of interest is included in the input endoscopic image, and does not output the information related to the region of interest in a case where the region of interest is not included in the input endoscopic image. Therefore, the learned model 631 functions as a region-of-interest presence or absence determination unit that determines whether or not the region of interest is included in the input endoscopic image. In a case where the region of interest is included in the input endoscopic image, the learned model 631 outputs the information related to the region of interest (the position and the accuracy probability) to the enlarged image generation unit 622 and the output mode determination unit 623.

Fig. 5 is an explanatory diagram related to the learned model 631 (a region-of-interest learning model). The learned model 631 is, for example, a model that performs object detection such as regions with convolutional neural network (RCNN), Fast RCNN, Faster RCNN, single shot multibook detector (SSD), You Only Look Once (YOLO), or a neural network (NN) with a function of a segmentation network. In a case where the learned model 631 is constituted by a neural network including a convolutional neural network (CNN) that extracts a feature amount of an image, such as an RCNN, the input layer included in the learned model 631 includes a plurality of neurons that receive an input of a pixel value of an endoscopic image, and passes the input pixel value to the intermediate layer. The intermediate layer includes a plurality of neurons that extract an image feature amount of the endoscopic image, and passes the extracted image feature amount to the output layer. The output layer includes one or a plurality of neurons that output the region-of-interest information including the position of the region of interest and the like, and outputs the position of the region of interest and the accuracy probability (the estimated score) on the basis of the image feature amount output from the intermediate layer. Alternatively, the learned model 631 may input the image feature amount output from the intermediate layer to a support vector machine (SVM) to perform object recognition. The neural network (the learned model 631) learned using training data is assumed to be used as a program module that is a part of artificial intelligence software. The learned model 631 is used in the information processing device 6 including the control unit 62 (a CPU or the like) and the storage unit 63 as described above, and is performed by the information processing device 6 with arithmetic processing capability, so that the neural network system is configured. That is, the control unit 62 of the information processing device 6 performs an arithmetic operation of extracting the feature amount of the endoscopic image input to the input layer in accordance with a command from the learned model 631 stored in the storage unit 63, and outputs the position of the region of interest and the accuracy probability (the estimated score) from the output layer.

When acquiring the information related to the region of interest from the learned model 631, the enlarged image generation unit 622 extracts (cuts out) a partial region of the endoscopic image including the region of interest on the basis of the position of the region of interest included in the information. The enlarged image generation unit 622 generates an enlarged image in which the portion of the endoscopic image including the region of interest is enlarged by enlarging and displaying the extracted (cut out) partial region. The enlarged image generation unit 622 may generate the enlarged image of the region of interest using an electronic zoom (digital zoom) method, for example. By the electronic zoom (digital zoom), a part of the endoscopic image (the partial region of the endoscopic image including the region of interest) can be cut out and complementarily enlarged, and the enlarged image can be efficiently generated by software processing. For example, the enlarged image generation unit 622 generates the enlarged image on the basis of the enlargement ratio included in the preset data stored in the storage unit 63. The enlargement ratio may be a fixed value such as five times, or may be determined depending on the number of pixels of the partial region of the endoscopic image including the extracted (cut out) region of interest. In a case where the enlargement ratio is determined depending on the number of pixels, the enlargement ratio may be determined on the basis of an inverse proportion coefficient that decreases the enlargement ratio as the number of pixels increases. The enlarged image generation unit 622 may generate the enlarged image by superimposing the accuracy probability included in the information related to the region of interest on the enlarged image. The enlarged image generation unit 622 outputs the generated enlarged image to the output unit 624.

When acquiring the information related to the region of interest from the learned model 631, the output mode determination unit 623 determines an output mode (a display mode) when the enlarged image is output (displayed) on the basis of the accuracy probability of the region of interest included in the information. The output mode (the display mode) includes, for example, a sub-screen display mode in which the enlarged image is displayed on a screen (a sub-screen) different from the screen (a main screen) on which the endoscopic image is displayed, and a main screen switching display mode in which the enlarged image is displayed by switching from the endoscopic image to the enlarged image on the screen (the main screen) on which the endoscopic image is displayed. In a case where the accuracy probability of the region of interest output from the learned model 631 is less than a predetermined value, the output mode determination unit 623 determines the output mode (the display mode) when the enlarged image is output (displayed) as the sub-screen display mode. When the accuracy probability of the region of interest output from the learned model 631 is equal to or larger than the predetermined value, the output mode determination unit 623 determines the output mode (the display mode) when the enlarged image is output (displayed) as the main screen switching display mode or the sub-screen display mode on the basis of the set value (the preset value for determining the output mode) included in the preset data stored in the storage unit 63.

The preset data stored in the storage unit 63 includes a predetermined value of the accuracy probability used when the output mode is determined (an accuracy probability threshold for classifying the accuracy probability of the region of interest) and a set value (a preset value) for determining the output mode in a case where the accuracy probability is equal to or larger than the predetermined value. In addition, the preset data includes a flag value indicating whether or not to change to the special light observation mode at the time of enlargement and display. When acquiring the information related to the region of interest from the learned model 631, the output mode determination unit 623 determines whether or not to change a white light observation mode to the special light observation mode at the time of enlargement and display on the basis of the flag value included in the preset data. The output mode determination unit 623 generates output mode data including the output mode determined as described above and the flag value of the observation mode (necessity of change to the special light observation mode), and outputs the generated output mode data to the output unit 624. The output mode data is data for controlling an output mode when an enlarged image is displayed, and includes, for example, a set value (a preset value) indicating whether the screen is set as a main screen or a sub-screen, and a flag value indicating whether or not to change to the special light observation mode.

The output unit 624 outputs the enlarged image acquired from the enlarged image generation unit 622 and the output mode data acquired from the output mode determination unit 623 to the processor 20 for an endoscope. On the basis of the enlarged image and the output mode data output from the information processing device 6 (the output unit 624), the processor 20 for an endoscope causes the display device 50 to display the enlarged image either in the output mode of the main screen or in the output mode of the sub-screen. In a case where it is necessary to change to the special light observation mode on the basis of the flag value of the observation mode included in the output mode data, the processor 20 for an endoscope changes to the special light observation mode such as narrow band imaging (NBI) when the enlarged image is displayed.

In the present embodiment, the functional units in a series of processing have been described while being divided into functional units implemented by the control unit 21 of the processor 20 for an endoscope and functional units implemented by the control unit 62 of the information processing device 6, but the division of these functional units is an example and is not limited thereto. The control unit 21 of the processor 20 for an endoscope may function as all the functional units implemented by the control unit 62 of the information processing device 6. That is, the processor 20 for an endoscope may substantially include the information processing device 6. Alternatively, the control unit 21 of the processor 20 for an endoscope may only output the captured image captured by the image sensor, and the control unit 62 of the information processing device 6 may function as all the functional units that perform the subsequent processing. Alternatively, the control unit 21 of the processor 20 for an endoscope and the control unit 62 of the information processing device 6 may perform, for example, inter-process communication, thereby functioning in cooperation as the functional units in the series of processing.

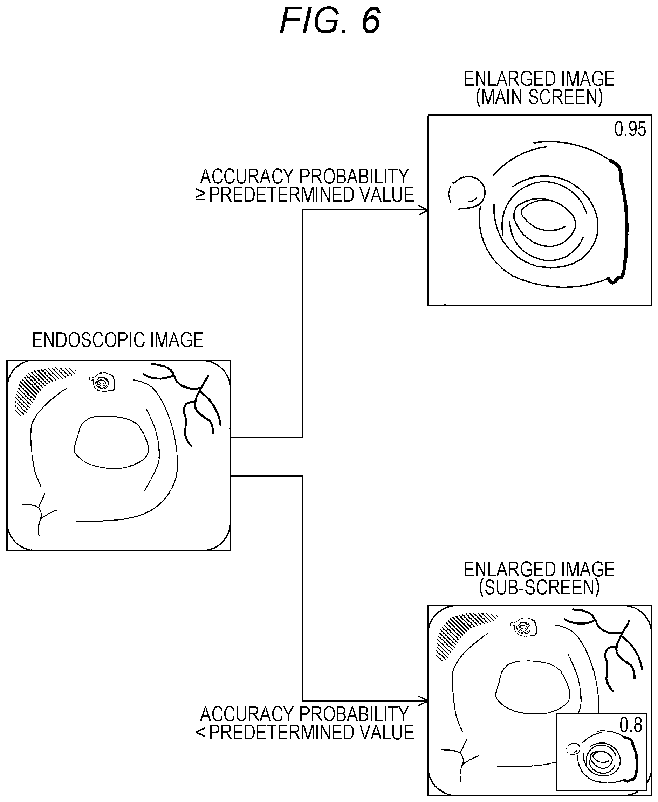

Fig. 6 is an explanatory diagram illustrating an aspect of a display screen of an enlarged image. In the present embodiment, the left screen is a main screen on which an endoscopic image is displayed. The upper right screen is a screen showing a state where the enlarged image in which the region of interest is enlarged is displayed on the main screen, and is a display mode based on the main screen switching display mode. The lower right screen is a screen showing a state where the enlarged image in which the region of interest is enlarged is displayed on the sub-screen, and is a display mode based on the sub-screen display mode.

The accuracy probability (the estimated score) of the region of interest is superimposed on these enlarged images. In a case where the accuracy probability is less than a predetermined value such as 0.9, the enlarged image is displayed on the sub-screen. In a case where the accuracy probability is equal to or larger than the predetermined value, the enlarged image is displayed on the main screen or the sub-screen on the basis of the set value (the preset value) included in the preset data.

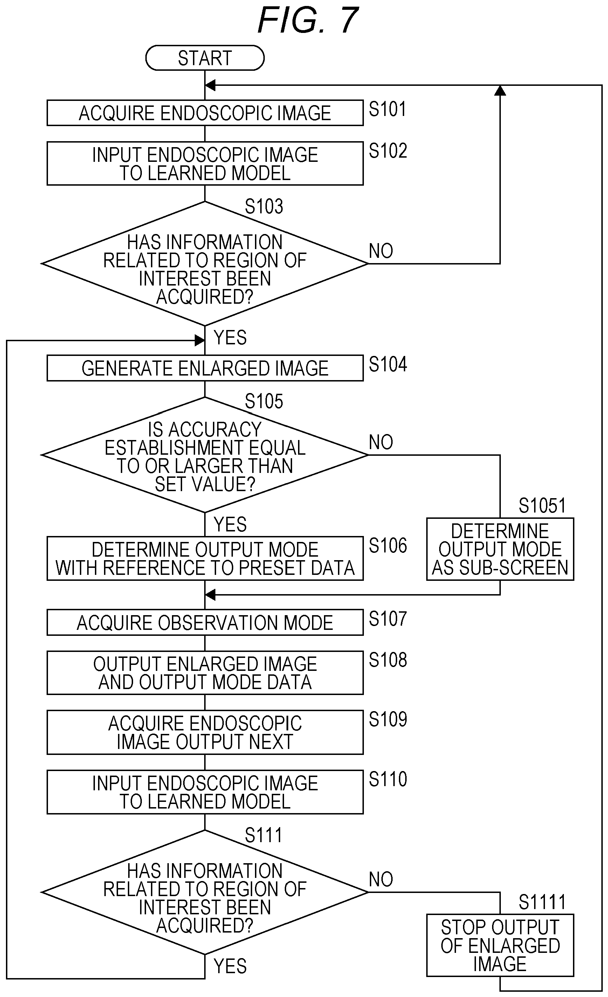

Fig. 7 is a flowchart illustrating an example of a processing procedure performed by the control unit 62 of the information processing device 6. For example, the information processing device 6 starts processing of the flowchart on the basis of contents input from the input unit 8 connected to the information processing device 6.

The control unit 62 of the information processing device 6 acquires an endoscopic image or the like output from the processor 20 for an endoscope (S101). The control unit 62 of the information processing device 6 may acquire an endoscopic image from the processor 20 for an endoscope in synchronization with the start of capturing of the body cavity by the processor 20 for an endoscope. The endoscopic image acquired by the control unit 62 of the information processing device 6 from the processor 20 for an endoscope may be a still image or a video.

The control unit 62 of the information processing device 6 inputs the endoscopic image to the learned model 631 (S102). The learned model 631 to which the endoscopic image is input outputs the position and the accuracy probability as the information related to the region of interest in a case where the region of interest is included in the input endoscopic image, and does not output the information related to the region of interest in a case where the region of interest is not included in the input endoscopic image.

The control unit 62 of the information processing device 6 determines whether or not the information related to the region of interest has been acquired from the learned model 631 (S103). If the information related to the region of interest has not been acquired from the learned model 631 (S103: NO), the control unit 62 of the information processing device 6 performs loop processing to perform the processing of S101 again.

If the information related to the region of interest has been acquired from the learned model 631 (S103: YES), the control unit 62 of the information processing device 6 generates an enlarged image of the region of interest on the basis of the position of the region of interest included in the information related to the region of interest (S104). The control unit 62 of the information processing device 6 extracts (cuts out) a partial region of the endoscopic image including the region of interest on the basis of the position of the region of interest, and generates the enlarged image of the region of interest using, for example, an electronic zoom (digital zoom) method. The control unit 62 of the information processing device 6 may associate the generated enlarged image of the region of interest with the endoscopic image including the region of interest, and store these images in the storage unit 63.

The control unit 62 of the information processing device 6 determines whether or not the accuracy establishment of the region of interest included in the information related to the region of interest is equal to or larger than a set value (S105). The control unit 62 of the information processing device 6 refers to a predetermined value (an accuracy probability threshold for classifying the accuracy establishment of the region of interest) included in the preset data stored in the storage unit 63, and determines whether or not the acquired accuracy probability is equal to or larger than the set value. If the accuracy establishment of the region of interest is not equal to or larger than the set value (S105: NO), that is, if the accuracy establishment of the region of interest is less than the set value, the control unit 62 of the information processing device 6 determines the output mode as the sub-screen.

If the accuracy establishment of the region of interest is equal to or larger than the set value (S105: YES), the control unit 62 of the information processing device 6 determines the output mode with reference to the pre data (S106). The control unit 62 of the information processing device 6 determines whether the enlarged image is displayed on the main screen or the sub-screen (the output mode) on the basis of the set value (the preset value) included in the preset data. In the present embodiment, if the accuracy establishment of the region of interest is equal to or larger than the set value, the output mode is determined with reference to the preset data, but it is not limited thereto. For example, the control unit 62 of the information processing device 6 may display a selection screen for selecting the output mode as a pop-up screen on the display unit 7 connected to the information processing device 6, and determine the output mode on the basis of a selection operation by the operator of the endoscope, which is input from the input unit 8.

The control unit 62 of the information processing device 6 acquires an observation mode at the time of enlargement and display with reference to the preset data (S107). The control unit 62 of the information processing device 6 determines the observation mode at the time of enlargement and display on the basis of the flag value of the observation mode (necessity of change to the special light observation mode) included in the preset data.

The control unit 62 of the information processing device 6 outputs the enlarged image and the output mode data (S108). The output mode data includes an output mode (a display mode) and an observation mode (change to the special light observation mode) when the enlarged image is displayed. The control unit 62 of the information processing device 6 outputs the enlarged image and the output mode data to the processor 20 for an endoscope. On the basis of the enlarged image and the output mode data output from the information processing device 6 (the output unit 624), the processor 20 for an endoscope causes the display device 50 to display the enlarged image either in the output mode of the main screen or in the output mode of the sub-screen. In a case where it is necessary to change to the special light observation mode on the basis of the flag value of the observation mode included in the output mode data, the processor 20 for an endoscope changes to the special light observation mode such as narrow band imaging (NBI) when the enlarged image is displayed.

The control unit 62 of the information processing device 6 acquires an endoscopic image output next from the processor 20 for an endoscope (S109). Endoscopic images are sequentially output from the processor 20 for an endoscope, and the control unit 62 of the information processing device 6 sequentially acquires the endoscopic images.

The control unit 62 of the information processing device 6 inputs the endoscopic image to the learned model 631 (S110). The control unit 62 of the information processing device 6 determines whether or not the information related to the region of interest has been acquired from the learned model 631 (S111). The control unit 62 of the information processing device 6 performs the processing of S110 and S111 similarly to the processing of S102 and S103.

If the information related to the region of interest has not been acquired from the learned model 631 (S111: NO), the control unit 62 of the information processing device 6 stops the output of the enlarged image. By stopping the output of the enlarged image, the image displayed on the main screen is switched from the enlarged image to the endoscopic image. In a case where the enlarged image is displayed on the generated sub-screen, the sub-screen is closed. After stopping the output of the enlarged image, the control unit 62 of the information processing device 6 performs loop processing to perform the processing of S101 again. If the information related to the region of interest has been acquired from the learned model 631 (S111: YES), the control unit 62 of the information processing device 6 performs loop processing to perform the processing of S104 again.

According to the present embodiment, the series of processing is performed by the control unit 62 of the information processing device 6, but it is not limited thereto. The series of processing may be performed by the control unit 21 of the processor 20 for an endoscope. Alternatively, the series of processing may be performed in cooperation between the control unit 21 of the processor 20 for an endoscope and the control unit 62 of the information processing device 6, for example, by performing inter-process communication. In the present embodiment, the control unit 62 of the information processing device 6 outputs the generated enlarged image to the processor 20 for an endoscope, but it is not limited thereto, and the control unit 62 of the information processing device 6 may output the enlarged image to the display unit 7 connected to the information processing device 6 and display the enlarged image on the display unit 7.

According to the present embodiment, the information processing device 6 inputs an image (an endoscopic image) captured by an endoscope to the learned model 631, and acquires the position of the region of interest output from the learned model 631 in a case where the region of interest (ROI) is included in the image (the endoscopic image). The information processing device 6 extracts a partial region of the endoscopic image including the region of interest on the basis of the acquired position of the region of interest, enlarges and displays the partial region, and outputs an enlarged image in which the portion of the endoscopic image including the region of interest is enlarged. As a result, in a case where the region of interest (ROI) is included in the endoscopic image, the processing of enlarging and displaying the region of interest can be efficiently performed, and the visibility of the region of interest by the operator of the endoscope can be improved by the enlarged image generated by performing the processing of enlarging and displaying. Since the electronic zoom operation for enlargement and display is automatically performed on the basis of the presence or absence of the region of interest, the zoom operation by the operator of the endoscope can be made unnecessary, and the operability of the endoscope can be improved. Since the region of interest such as a lesion displayed in an enlarged manner can be efficiently viewed, it is expected to facilitate a determination by the operator of the endoscope such as a doctor and reduce treatments such as biopsy, for example.

According to the present embodiment, the information processing device 6 determines an output mode when the enlarged image is output on the basis of the accuracy probability of the region of interest acquired from the learned model 631, and outputs the enlarged image in the output mode. The output mode includes an output mode in which the enlarged image is displayed on a screen (a sub-screen) different from the screen on which the endoscopic image is displayed, and an output mode of switching from the endoscopic image to the enlarged image on the screen (a main screen) on which the endoscopic image is displayed. In a case where the accuracy probability is less than a predetermined value, by using the output mode of outputting the enlarged image so that the enlarged image is displayed on the screen (the sub-screen) different from the screen on which the endoscopic image is displayed, the information processing device 6 outputs the enlarged image of the region of interest so that the enlarged image is displayed on the different screen (the sub-screen) while maintaining the state of the screen (the main screen) on which the endoscopic image is displayed. As a result, when it is estimated (output) by the learned model 631 that the accuracy probability of the region of interest is less than the predetermined value, the state of the screen (the main screen) on which the endoscopic image is displayed is maintained, so that it is possible for the operator of the endoscope to continue viewing the endoscopic image.

For the region of interest in which the accuracy probability is equal to or larger than the predetermined value, by using the output mode in which the screen (the main screen) on which the endoscopic image is displayed is switched from the endoscopic image to the enlarged image of the region of interest, the visibility of the enlarged image (the region of interest in which the accuracy probability is equal to or larger than the predetermined value) by the operator of the endoscope can be improved. Even in the region of interest in which the accuracy probability is equal to or larger than the predetermined value, control may be executed with a preset value to output the enlarged image of the region of interest to be displayed on the different screen (the sub-screen) while maintaining the state of the screen (the main screen) on which the endoscopic image is displayed, similarly to the region of interest in which the accuracy probability is less than the predetermined value. That is, the output mode when the enlarged image of the region of interest whose accuracy probability is equal to or larger than the predetermined value includes the output mode of switching to the screen (the main screen) on which the endoscopic image is displayed and the output mode of outputting (displaying) the enlarged image on the screen (the sub-screen) different from the screen (the main screen) on which the endoscopic image is displayed, and a plurality of these output modes can be selectively used. The set value (the preset value) for determining the output mode to be selectively used is stored in a storage area accessible from the control unit 62 of the information processing device 6, such as the storage unit 63 of the information processing device 6, and the information processing device 6 determines the output mode when the enlarged image of the region of interest whose accuracy probability is equal to or larger than the predetermined value is input on the basis of the set value, so that the availability can be improved based on the operator of the endoscope.

According to the present embodiment, when outputting the enlarged image of the region of interest, the information processing device 6 outputs a signal to change the observation mode of the endoscope to the special light observation mode to the endoscope (an endoscope processor). Therefore, it is possible to automatically change the observation mode from the white light observation mode to the special light observation mode with the presence of the region of interest in the endoscopic image as a trigger, and to efficiently provide the operator of the endoscope with the enlarged image of the region of interest illuminated with special light such as narrow band imaging (NBI).

According to the present embodiment, the information processing device 6 stops the output of the enlarged image in a case where the region of interest is no longer included in the endoscopic image, that is, in a case where the information related to the region of interest (the position and the accuracy probability) is not output from the learned model 631. In a case where the enlarged image is displayed on the screen (the sub-screen) different from the screen (the main screen) on which the endoscopic image is displayed, by stopping the output of the enlarged image, the sub-screen is closed. Alternatively, the sub-screen may transition to a non-display state, an inactive state, or a minimized state. In a case where the screen (the main screen) on which the endoscopic image is displayed is switched from the endoscopic image to the enlarged image and the enlarged image is displayed, by stopping the output of the enlarged image, the image displayed on the main screen is switched from the enlarged image to the original endoscopic image. In a case where the special light observation mode is used to display the enlarged image, when the region of interest is no longer included in the endoscopic image, the observation mode may transition from the special light observation mode to the white light observation mode. In a case where the region of interest is not included in the endoscopic image acquired after the enlarged image is output as described above, by automatically returning the output mode (the display mode) when the enlarged image is output (displayed) to the original output mode (the display mode) of outputting (displaying) only the endoscopic image with the absence of the region of interest as a trigger, it is possible to reduce the labor of the operation by the operator of the endoscope and improve the operability.

### (Second Embodiment)

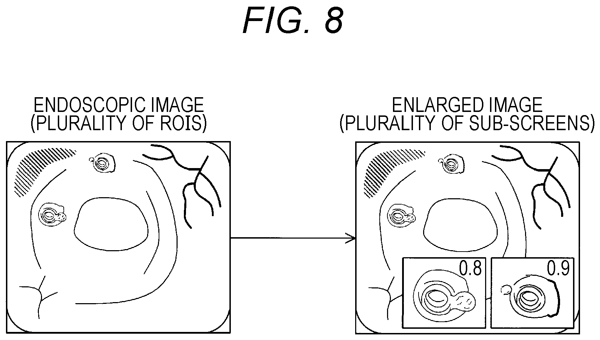

Fig. 8 is an explanatory diagram illustrating an aspect of a display screen of an enlarged image according to a second embodiment (a plurality of regions of interest). When acquiring information related to a plurality of regions of interest (positions and accuracy probabilities) from an endoscopic image, the control unit 62 (the learned model 631) of the information processing device 6 individually outputs a plurality of enlarged images obtained by individually enlarging the plurality of regions of interest. In the present embodiment, the left screen is a main screen on which an endoscopic image is displayed, and the endoscopic image includes a plurality of regions of interest.

The control unit 62 of the information processing device 6 may generate sub-screens (screens different from the screen on which the endoscopic image is displayed), the number of which is equal to the number of regions of interest, and display the enlarged images on the generated sub-screens. Alternatively, when outputting (displaying) the generated plurality of enlarged images, the control unit 62 of the information processing device 6 may generate output mode data in an output mode (a display mode) of displaying the enlarged image with the highest accuracy establishment among the plurality of enlarged images on the main screen and outputting (displaying) other enlarged images on the individual sub-screens, and output the output mode data to the processor 20 for an endoscope.

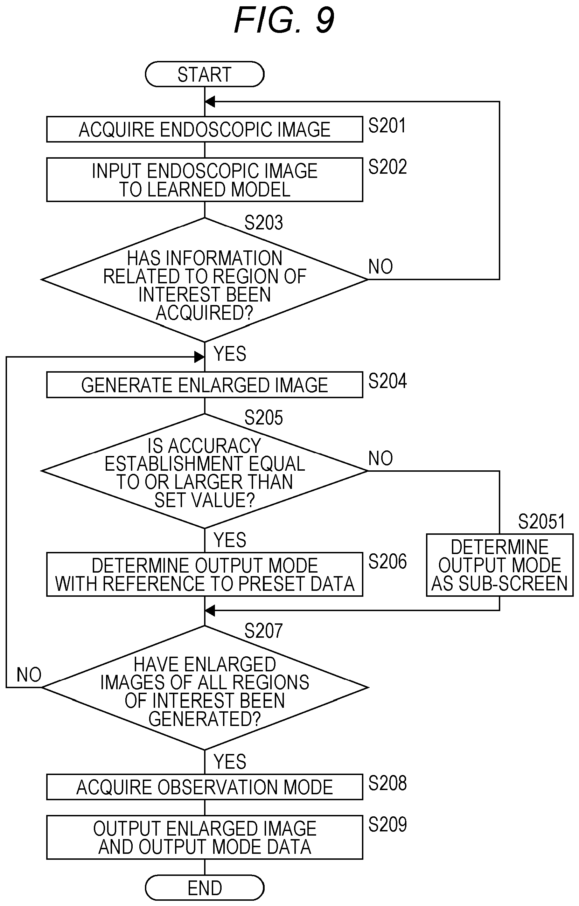

Fig. 9 is a flowchart illustrating an example of a processing procedure performed by the control unit 62 of the information processing device 6. The control unit 62 of the information processing device 6 performs processing from S201 to S206 and S2061 similarly to the processing from S101 to S106 and S1051 in the first embodiment.

The control unit 62 of the information processing device 6 determines whether or not enlarged images of all the regions of interest have been generated (S207). In a case where a plurality of regions of interest are included in an endoscopic image, the learned model 631 outputs information (information related to the region of interest) including the position and the accuracy probability of each of the plurality of regions of interest. The control unit 62 of the information processing device 6 may temporarily store the positions and the accuracy probabilities of the plurality of regions of interest output from the learned model 631 in the storage unit 63 in, for example, an array format, and process the positions and the accuracy probabilities in the order of sequence numbers determined by the number of regions of interest included in the endoscopic image. For example, in a case where the enlarged images of the regions of interest corresponding to all the sequence numbers are generated, that is, in a case where there is no unprocessed region of interest, the control unit 62 of the information processing device 6 determines that the enlarged images of all the regions of interest have been generated. If the enlarged images of all the regions of interest have not been generated (S207: NO), the control unit 62 of the information processing device 6 performs loop processing to perform the processing of S204 again.

If the enlarged images of all the regions of interest have been generated (S207: YES), the control unit 62 of the information processing device 6 performs processing of S208 and S209 similarly to the processing of S107 and S108 in the first embodiment. When outputting (displaying) the generated plurality of enlarged images to the processor 20 for an endoscope, the control unit 62 of the information processing device 6 may output the output mode data to the processor 20 for an endoscope in an output mode (a display mode) of outputting (displaying) each of the plurality of enlarged image on each of the sub-screens. Alternatively, when outputting (displaying) the generated plurality of enlarged images, the control unit 62 of the information processing device 6 may output the output mode data to the processor 20 for an endoscope in an output mode (a display mode) of displaying the enlarged image with the highest accuracy establishment among the plurality of enlarged images on the main screen and outputting (displaying) other enlarged images on the individual sub-screens. Similarly to the first embodiment, the control unit 62 of the information processing device 6 may stop the output of the enlarged image in a case where the region of interest is no longer included in the acquired endoscopic image.

According to the present embodiment, when acquiring the information (positions and accuracy probabilities) related to a plurality of regions of interest from a single endoscopic image such as one frame in a video, the information processing device 6 outputs each of a plurality of enlarged images in which the plurality of regions of interest are enlarged to the processor 20 for an endoscope. In outputting each of the plurality of enlarged images, the information processing device 6 may generate sub-screens (screens different from the screen on which the endoscopic image is displayed), the number of which is equal to the number of regions of interest, and display the individual enlarged images on the individual generated sub-screens. Even in a case where a plurality of regions of interest are included in the endoscopic image, the enlarged images of the plurality of regions of interest are displayed on the individual sub-screens, so that it is possible to efficiently provide information related to the region of interest to the operator of the endoscope.

### (Third Embodiment)

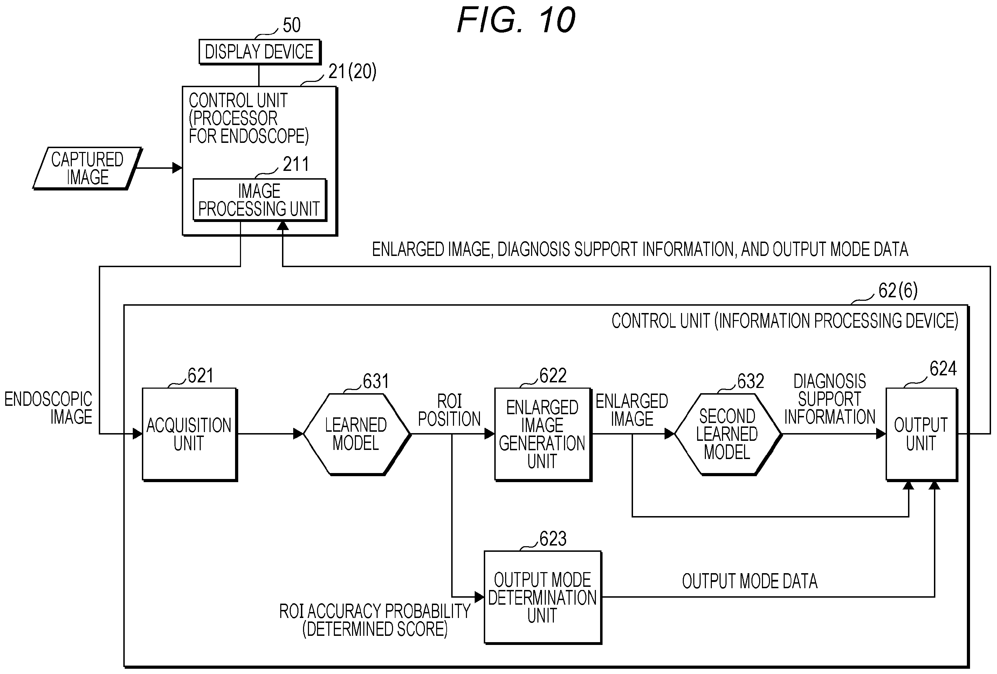

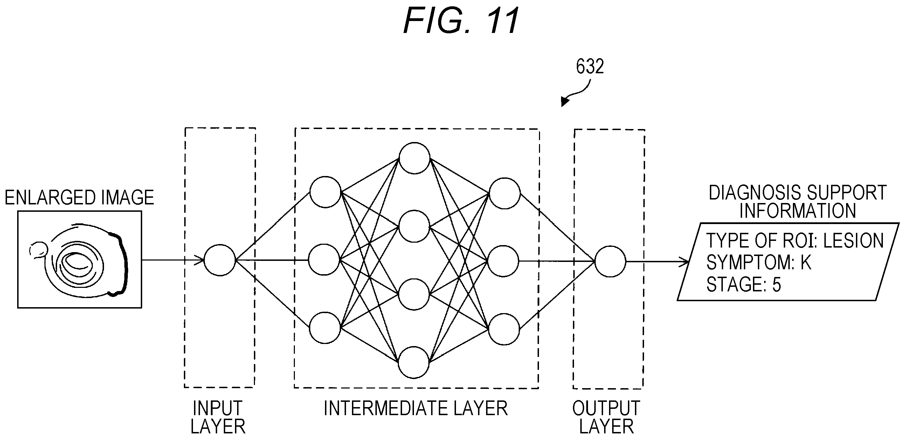

Fig. 10 is a functional block diagram exemplifying functional units included in the control unit 62 of an information processing device 6 according to a third embodiment (a second learned model 632). Fig. 11 is an explanatory diagram related to the second learned model 632 (a diagnosis support learning model). The information processing device 6 according to the third embodiment further includes the second learned model 632. The storage unit 63 of the information processing device 6 according to the third embodiment stores an entity file (an instance file of a neural network (NN)) constituting the second learned model 632 (the diagnosis support learning model). These entity files may be configured as a part of the program.

As in the first embodiment, the control unit 62 of the information processing device 6 according to the third embodiment executes the program stored in the storage unit 63 to function as the acquisition unit 621, the learned model 631, the enlarged image generation unit 622, the output mode determination unit 623, and the output unit 624, and further function as the second learned model 632. The acquisition unit 621, the learned model 631, the enlarged image generation unit 622, and the output mode determination unit 623 perform processing similar to that of the first embodiment. Furthermore, the enlarged image generation unit 622 outputs a generated enlarged image to the second learned model 632.

The second learned model 632 includes a neural network similarly to the learned model 631, and is, for example, a convolution neural network (CNN). The second learned model 632 is learned to output diagnosis support information related to a region of interest in a case where an enlarged image including the region of interest is input. The diagnosis support information related to the region of interest may include, for example, the type of the region of interest such as a lesion, a lesion candidate, a drug, a treatment tool, or a marker, or the classification and stage of the lesion. The second learned model 632 is a model that outputs diagnosis support information including the type of the region of interest and the like on the basis of the input enlarged image (the enlarged image including the region of interest), and corresponds to a diagnosis support learning model. The second learned model 632 outputs the diagnosis support information output (estimated) on the basis of the input enlarged image to the output unit 624.

Similarly to the first embodiment, the output unit 624 outputs the enlarged image acquired from the enlarged image generation unit 622, the output mode data acquired from the output mode determination unit 623, and the diagnosis support information acquired from the second learned model 632 (the diagnosis support learning model) to the processor 20 for an endoscope. On the basis of the enlarged image, the diagnosis support information, and the output mode data output from the information processing device 6 (the output unit 624) as in the first embodiment, the processor 20 for an endoscope causes the display device 50 to display the enlarged image and the diagnosis support information in either the output mode of the main screen or the output mode of the sub-screen.

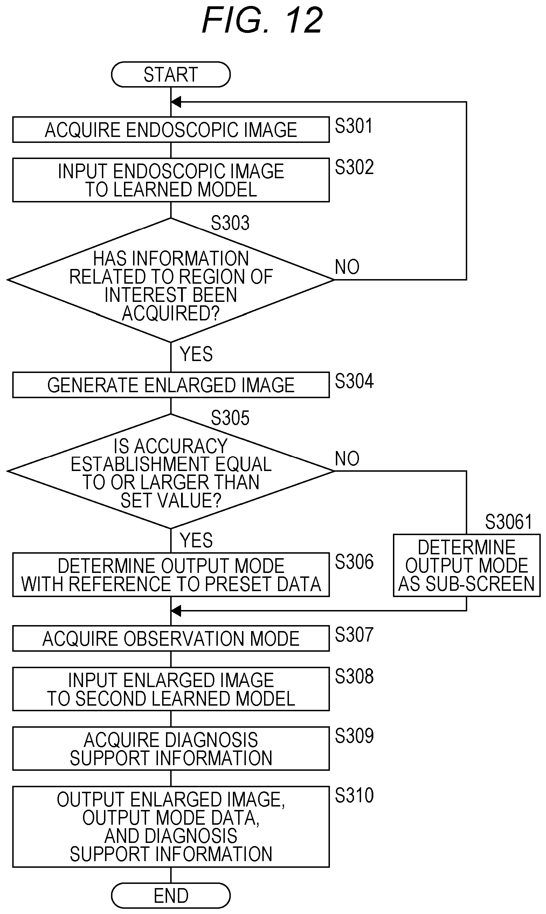

Fig. 12 is a flowchart illustrating an example of a processing procedure performed by the control unit 62 of the information processing device 6. The control unit 62 of the information processing device 6 performs processing from S301 to S307 similarly to the processing from S101 to S107 in the first embodiment.

The control unit 62 of the information processing device 6 inputs an enlarged image to the second learned model 632 (S308). The control unit 62 of the information processing device 6 acquires diagnosis support information from the second learned model 632 (S309). The second learned model 632 to which the enlarged image is input outputs diagnosis support information related to a region of interest. The diagnosis support information related to the region of interest includes, for example, the type of the region of interest such as a lesion, a lesion candidate, a drug, a treatment tool, or a marker, or the classification and stage of the lesion.

The control unit 62 of the information processing device 6 outputs the enlarged image, output mode data, and the diagnosis support information (S310). The control unit 62 of the information processing device 6 outputs the enlarged image, the output mode data, and the diagnosis support information to the processor 20 for an endoscope as in the first embodiment. On the basis of the enlarged image, the diagnosis support information, and the output mode data output from the information processing device 6 (the output unit 624) as in the first embodiment, the processor 20 for an endoscope causes the display device 50 to display the enlarged image and the diagnosis support information in either the output mode of the main screen or the output mode of the sub-screen.

According to the present embodiment, when the learned model 631 outputs information (a position and an accuracy probability) related to the region of interest, the information processing device 6 inputs the enlarged image of the region of interest to the second learned model 632, and acquires diagnosis support information (the type of the region of interest, the classification and stage of a lesion, and the like) related to the region of interest from the second learned model 632. Since the image input to the second learned model 632 is the enlarged image of the region of interest extracted from the endoscopic image, the information amount ratio of the region of interest in the enlarged image can be increased as compared with the information amount ratio of the region of interest in the endoscopic image, and the estimation accuracy of the second learned model 632 can be improved. By associating the enlarged image of the region of interest with the diagnosis support information output (estimated) by the second learned model 632 on the basis of the enlarged image, and outputting and displaying the enlarged image and the diagnosis support information on the sub-screen or the like, the information processing device 6 can efficiently provide the operator of the endoscope with the enlarged image of the region of interest and the diagnosis support information.

### Reference Signs List

- S: Diagnosis support system
- 10: Endoscope device
- 15: Keyboard
- 16: Storage rack
- 20: Processor for an endoscope
- 21: Control unit
- 211: Image processing unit
- 22: Main storage device
- 23: Auxiliary storage device
- 24: Communication unit
- 25: Touch panel
- 26: Display device I/F
- 27: Input device I/F
- 28: Reading unit
- 31: Endoscope connector
- 311: Electrical connector
- 312: Optical connector
- 33: Light source
- 34: Pump
- 35: Water supply tank
- 36: Air/water supply port
- 40: Endoscope
- 43: Operation unit
- 431: Control button
- 433: Bending knob
- 44: Insertion portion (Flexible tube)
- 441: Soft portion
- 442: Bending section
- 443: Distal tip portion
- 444: Image-capturing unit
- 45: Bend preventing portion
- 48: Scope connector
- 49: Universal cord
- 50: Display device
- 6: Information processing device
- 61: Communication unit
- 62: Control unit
- 621: Acquisition unit
- 622: Enlarged image generation unit
- 623: Output mode determination unit
- 624: Output unit
- 63: Storage unit
- 631: Learned model (Region-of-interest learning model)
- 632: Second learned model (Diagnosis support learning model)
- 64: Input/output I/F
- 7: Display unit
- 8: Input unit

## Claims

1. A computer-implemented information processing method causing a computer to perform processing of:
acquiring an image captured by an endoscope (40);
inputting the acquired image to a learned model (631), the learned model (631) being learned to output a position of a region of interest included in the image in a case where the image captured by the endoscope (40) is input;
acquiring a position of a region of interest included in the acquired image from the learned model (631), wherein the position includes coordinates of two points in an image coordinate system of the image;
extracting a partial region of the image including the region of interest on the basis of the position of the region of interest; and
generating an enlarged image in which a portion of the image including the region of interest is enlarged by enlarging the extracted partial region using an electronic zoom;
outputting the enlarged image to a second learned model (632),
wherein the second learned model (632) is learned to output diagnosis support information related to the region of interest in a case where the enlarged image including the region of interest is input to the second learned model (632) , wherein the diagnosis support information includes a type of the region of interest,
wherein the method further comprises
acquiring the diagnosis support information related to the region of interest from the second learned model (632), and
outputting the enlarged image and the acquired diagnosis support information by associating the enlarged image with the acquired diagnosis support information .

2. The method according to claim 1, wherein
in a case where an image captured by the endoscope (40) is input, the learned model (631) outputs a position and an accuracy probability of the region of interest included in the image,
wherein the method further comprises, in a case where the accuracy probability is less than a predetermined value, outputting the enlarged image so that the enlarged image is displayed on a screen different from a screen on which the acquired image is displayed, and
in a case where the accuracy probability is equal to or larger than the predetermined value, outputting the enlarged image so that the acquired image is switched to the enlarged image on a screen on which the acquired image is displayed, or the enlarged image is displayed on a screen different from the screen on which the acquired image is displayed.

3. The method according to claim 2, wherein
a set value for determining an output mode of an enlarged image in a case where the accuracy probability is equal to or larger than the predetermined value is stored in advance, and
the enlarged image in a case where the accuracy probability is equal to or larger than the predetermined value is output in an output mode based on the set value.

4. The method according to any one of claims 1 to 3, wherein
in a case where the enlarged image is output, information for changing an observation mode of the endoscope (40) to a special light observation mode is output.

5. The method according to any one of claims 1 to 4, wherein
in a case where positions of a plurality of the regions of interest included in the acquired image are acquired from the learned model (631), enlarged images, in each of which a portion of an image including each of the plurality of regions of interest is enlarged, are output on the basis of the acquired positions of the plurality of regions of interest.

6. A computer program comprising instructions which, when the program is executed by a computer, cause the computer to carry out the method of any one of claims 1 to 5.

7. An information processing device (6) comprising:
an image acquisition unit (621) configured to acquire an image captured by an endoscope (40);
an input unit (8) configured to inputs the acquired image to a learned model (631), the learned model (631) being learned to output a position of a region of interest included in the image in a case where the image captured by the endoscope (40) is input;
a position acquisition unit configured to acquire a position of a region of interest included in the acquired image from the learned model (631), wherein the position includes coordinates of two points in an image coordinate system of the endoscopic image;
an enlarged image generation unit (622) configured to extract a partial region of the image including the region of interest on the basis of the position of the region of interest,
generate an enlarged image in which a portion of the image including the region of interest is enlarged by enlarging the extracted partial region using an
electronic zoom, and
output the generated enlarged image to a second learned model (632),
wherein the second learned model (632) is learned to output diagnosis support information related to the region of interest in a case where the enlarged image including the region of interest is input to the second learned model (632), wherein the diagnosis support information includes a type of the region of interest,
wherein the information processing device further comprises
an output unit (624) configured to
acquire the diagnosis support information related to the region of interest from the second learned model (632), and
output the enlarged image and the acquired diagnosis support information by associating the enlarged image with the acquired diagnosis support information.

## Patentansprüche

1. Computerimplementiertes Informationsverarbeitungsverfahren, das einen Computer veranlasst, eine Verarbeitung durchzuführen zum:
Erfassen eines von einem Endoskop (40) aufgenommenen Bildes;
Eingeben des erfassten Bildes in ein gelerntes Modell (631), wobei das gelernte Modell (631) gelernt wird, eine Position eines in dem Bild enthaltenen Bereichs von Interesse in einem Fall auszugeben, in dem das von dem Endoskop (40) aufgenommene Bild eingegeben wird;
Erfassen einer Position eines in dem erfassten Bild enthaltenen Bereichs von Interesse aus dem gelernten Modell (631), wobei die Position Koordinaten von zwei Punkten in einem Bildkoordinatensystem des Bildes enthält;
Extrahieren eines Teilbereichs des Bildes, der den Bereich von Interesse enthält, auf der Basis der Position des Bereichs von Interesse; und
Erzeugen eines vergrößerten Bildes, in dem ein Abschnitt des Bildes, der den Bereich von Interesse enthält, vergrößert ist, durch Vergrößern des extrahierten Teilbereichs unter Verwendung eines elektronischen Zooms;
Ausgeben des vergrößerten Bildes an ein zweites gelerntes Modell (632),
wobei das zweite gelernte Modell (632) gelernt wird, Diagnoseunterstützungsinformationen in Bezug auf den Bereich von Interesse in einem Fall auszugeben, in dem das vergrößerte Bild, das den Bereich von Interesse enthält, in das zweite gelernte Modell (632) eingegeben wird, wobei die Diagnoseunterstützungsinformationen einen Typ des Bereichs von Interesse enthalten,
wobei das Verfahren ferner umfasst:
Erfassen der Diagnoseunterstützungsinformationen in Bezug auf den Bereich von Interesse aus dem zweiten gelernten Modell (632), und
Ausgeben des vergrößerten Bildes und der erfassten Diagnoseunterstützungsinformationen durch Verknüpfen des vergrößerten Bildes mit den erfassten Diagnoseunterstützungsinformationen.

2. Verfahren nach Anspruch 1, wobei
in einem Fall, in dem ein von dem Endoskop (40) aufgenommenes Bild eingegeben wird, das gelernte Modell (631) eine Position und eine Genauigkeitswahrscheinlichkeit des in dem Bild enthaltenen Bereichs von Interesse ausgibt,
wobei das Verfahren ferner in einem Fall, in dem die Genauigkeitswahrscheinlichkeit kleiner als ein vorbestimmter Wert ist, das Ausgeben des vergrößerten Bildes umfasst, so dass das vergrößerte Bild auf einem Bildschirm angezeigt wird, der sich von einem Bildschirm unterscheidet, auf dem das erfasste Bild angezeigt wird, und
in einem Fall, in dem die Genauigkeitswahrscheinlichkeit gleich oder größer als der vorbestimmte Wert ist, das Ausgeben des vergrößerten Bildes umfasst, so dass das erfasste Bild auf das vergrößerte Bild auf einem Bildschirm umgeschaltet wird, auf dem das erfasste Bild angezeigt wird, oder das vergrößerte Bild auf einem Bildschirm angezeigt wird, der sich von dem Bildschirm unterscheidet, auf dem das erfasste Bild angezeigt wird.

3. Verfahren nach Anspruch 2, wobei
ein eingestellter Wert zum Bestimmen eines Ausgabemodus eines vergrößerten Bildes in einem Fall, in dem die Genauigkeitswahrscheinlichkeit gleich oder größer als der vorbestimmte Wert ist, im Voraus gespeichert wird, und
das vergrößerte Bild in einem Fall, in dem die Genauigkeitswahrscheinlichkeit gleich oder größer als der vorbestimmte Wert ist, in einem Ausgabemodus basierend auf dem eingestellten Wert ausgegeben wird.

4. Verfahren nach einem der Ansprüche 1 bis 3, wobei
in einem Fall, in dem das vergrößerte Bild ausgegeben wird, Informationen zum Ändern eines Beobachtungsmodus des Endoskops (40) in einen Speziallichtbeobachtungsmodus ausgegeben werden.

5. Verfahren nach einem der Ansprüche 1 bis 4, wobei
in einem Fall, in dem Positionen einer Vielzahl der in dem erfassten Bild enthaltenen Bereichen von Interesse aus dem gelernten Modell (631) erfasst werden, vergrößerte Bilder, in denen jeweils ein Abschnitt eines Bildes, welches einen jeweiligen der Vielzahl von Bereichen von Interesse enthält, vergrößert ist, auf der Basis der erfassten Positionen der Vielzahl von Bereichen von Interesse ausgegeben werden.

6. Computerprogramm, umfassend Anweisungen, die, wenn das Programm von einem Computer ausgeführt wird, den Computer veranlassen, das Verfahren nach einem der Ansprüche 1 bis 5 auszuführen.

7. Informationsverarbeitungsvorrichtung (6), umfassend:
eine Bilderfassungseinheit (621), die eingerichtet ist, ein von einem Endoskop (40) aufgenommenes Bild zu erfassen;
eine Eingabeeinheit (8), die eingerichtet ist, das erfasste Bild in ein gelerntes Modell (631) einzugeben, wobei das gelernte Modell (631) gelernt wird, eine Position eines in dem Bild enthaltenen Bereichs von Interesse in einem Fall auszugeben, in dem das von dem Endoskop (40) aufgenommene Bild eingegeben wird;
eine Positionserfassungseinheit, die eingerichtet ist, eine Position eines in dem erfassten Bild enthaltenen Bereichs von Interesse aus dem gelernten Modell (631) zu erfassen, wobei die Position Koordinaten von zwei Punkten in einem Bildkoordinatensystem des endoskopischen Bildes enthält;
eine Vergrößertes-Bild-Erzeugungseinheit (622), die eingerichtet ist,
einen Teilbereich des Bildes, der den Bereich von Interesse enthält, auf der Basis der Position des Bereichs von Interesse zu extrahieren,
ein vergrößertes Bild zu erzeugen, in dem ein Abschnitt des Bildes, der den Bereich von Interesse enthält, vergrößert ist, durch Vergrößern des extrahierten Teilbereichs unter Verwendung eines elektronischen Zooms; und
elektronisch zu zoomen, und
das erzeugte vergrößerte Bild an ein zweites gelerntes Modell (632) auszugeben,
wobei das zweite gelernte Modell (632) gelernt wird, Diagnoseunterstützungsinformationen in Bezug auf den Bereich von Interesse in einem Fall auszugeben, in dem das vergrößerte Bild, das den Bereich von Interesse enthält, in das zweite gelernte Modell (632) eingegeben wird, wobei die Diagnoseunterstützungsinformationen einen Typ des Bereichs von Interesse enthalten,
wobei die Informationsverarbeitungsvorrichtung ferner umfasst:
eine Ausgabeeinheit (624), die eingerichtet ist,
die Diagnoseunterstützungsinformationen in Bezug auf den Bereich von Interesse aus dem zweiten gelernten Modell (632) zu erfassen, und
das vergrößerte Bild und die erfassten Diagnoseunterstützungsinformationen durch Verknüpfen des vergrößerten Bildes mit den erfassten Diagnoseunterstützungsinformationen auszugeben.

## Revendications

1. Procédé de traitement d'informations mis en oeuvre par ordinateur amenant un ordinateur à effectuer un traitement consistant à :
acquérir une image capturée par un endoscope (40) ;
entrer l'image acquise dans un modèle appris (631), le modèle appris (631) étant appris pour délivrer en sortie une position d'une région d'intérêt incluse dans l'image dans un cas où l'image capturée par l'endoscope (40) est entrée ;
acquérir une position d'une région d'intérêt incluse dans l'image acquise à partir du modèle appris (631), dans lequel la position inclut des coordonnées de deux points dans un système de coordonnées d'image de l'image ;
extraire une région partielle de l'image incluant la région d'intérêt sur la base de la position de la région d'intérêt ; et
générer une image agrandie dans laquelle une partie de l'image incluant la région d'intérêt est agrandie en agrandissant la région partielle extraite en utilisant un zoom électronique;
délivrer en sortie l'image agrandie dans un second modèle appris (632),
dans lequel le second modèle appris (632) est appris pour délivrer en sortie des informations de support de diagnostic relatives à la région d'intérêt dans un cas où l'image agrandie incluant la région d'intérêt est entrée dans le second modèle appris (632), dans lequel les informations de support de diagnostic incluent un type de la région d'intérêt,
dans lequel le procédé comprend en outre
acquérir les informations de support de diagnostic relatives à la région d'intérêt à partir du second modèle appris (632), et
délivrer en sortie l'image agrandie et les informations de support de diagnostic acquises en associant l'image agrandie aux informations de support de diagnostic acquises.

2. Procédé selon la revendication 1, dans lequel
dans un cas où une image capturée par l'endoscope (40) est entrée, le modèle appris (631) délivre en sortie une position et une probabilité de précision de la région d'intérêt incluse dans l'image,
dans lequel le procédé comprend en outre, dans un cas où la probabilité de précision est inférieure à une valeur prédéterminée, la délivrance en sortie de l'image agrandie de sorte que l'image agrandie soit affichée sur un écran différent d'un écran sur lequel l'image acquise est affichée, et
dans un cas où la probabilité de précision est égale ou supérieure à la valeur prédéterminée, la délivrance en sortie de l'image agrandie de sorte que l'image acquise soit commutée vers l'image agrandie sur un écran sur lequel l'image acquise est affichée, ou que l'image agrandie soit affichée sur un écran différent de l'écran sur lequel l'image acquise est affichée.

3. Procédé selon la revendication 2, dans lequel
une valeur définie pour déterminer un mode de sortie d'une image agrandie dans un cas où la probabilité de précision est égale ou supérieure à la valeur prédéterminée est stockée à l'avance, et
l'image agrandie dans un cas où la probabilité de précision est égale ou supérieure à la valeur prédéterminée est délivrée en sortie dans un mode de sortie sur la base de la valeur définie.

4. Procédé selon l'une quelconque des revendications 1 à 3, dans lequel
dans un cas où l'image agrandie est délivrée en sortie, des informations pour changer un mode d'observation de l'endoscope (40) en un mode d'observation de lumière spéciale sont délivrées en sortie.

5. Procédé selon l'une quelconque des revendications 1 à 4, dans lequel
dans un cas où des positions d'une pluralité des régions d'intérêt incluses dans l'image acquise sont acquises à partir du modèle appris (631), des images agrandies, dans chacune desquelles une partie d'une image incluant chacune de la pluralité de régions d'intérêt est agrandie, sont délivrées en sortie sur la base des positions acquises de la pluralité de régions d'intérêt.

6. Programme informatique comprenant des instructions qui, lorsque le programme est exécuté par un ordinateur, amènent l'ordinateur à réaliser le procédé selon l'une quelconque des revendications 1 à 5.

7. Dispositif de traitement d'informations (6) comprenant :
une unité d'acquisition d'image (621) configurée pour acquérir une image capturée par un endoscope (40) ;
une unité d'entrée (8) configurée pour entrer l'image acquise dans un modèle appris (631), le modèle appris (631) étant appris pour délivrer en sortie une position d'une région d'intérêt incluse dans l'image dans un cas où l'image capturée par l'endoscope (40) est entrée ;
une unité d'acquisition de position configurée pour acquérir une position d'une région d'intérêt incluse dans l'image acquise à partir du modèle appris (631), dans lequel la position inclut des coordonnées de deux points dans un système de coordonnées d'image de l'image endoscopique ;
une unité de génération d'image agrandie (622) configurée pour
extraire une région partielle de l'image incluant la région d'intérêt sur la base de la position de la région d'intérêt,
générer une image agrandie dans laquelle une partie de l'image incluant la région d'intérêt est agrandie en agrandissant la région partielle extraite en utilisant un zoom électronique ; et
zoom électronique, et
délivrer en sortie l'image agrandie générée dans un second modèle appris (632),
dans lequel le second modèle appris (632) est appris pour délivrer en sortie des informations de support de diagnostic relatives à la région d'intérêt dans un cas où l'image agrandie incluant la région d'intérêt est entrée dans le second modèle appris (632), dans lequel les informations de support de diagnostic incluent un type de la région d'intérêt,
dans lequel le dispositif de traitement d'informations comprend en outre une unité de sortie (624) configurée pour
acquérir les informations de support de diagnostic relatives à la région d'intérêt à partir du second modèle appris (632), et
délivrer en sortie l'image agrandie et les informations de support de diagnostic acquises en associant l'image agrandie aux informations de support de diagnostic acquises.
